Europäisches Patentamt

European Patent Office

Office européen des brevets

(19)

(11) Numéro de publication: **0 047 204**
**B1**

(12) # FASCICULE DE BREVET EUROPEEN

(45) Date de publication du fascicule du brevet:
**21.09.83**

(51) Int. Cl.³: **C 07 C 29/84,** C 07 C 29/82,
B 01 D 3/36

(21) Numéro de dépôt: **81401322.3**

(22) Date de dépôt: **19.08.81**

(54) **Procédé de production d'alcools deshydratés utilisables comme composants d'un carburant pour moteur.**

(30) Priorité: **01.09.80 FR 8018901**

(43) Date de publication de la demande:
**10.03.82 Bulletin 82/10**

(45) Mention de la délivrance du brevet:
**21.09.83 Bulletin 83/38**

(84) Etats contractants désignés:
**BE CH DE GB IT LI NL SE**

(56) Documents cités:
**FR-A- 954 313**
**GB-A- 811 400**
**US-A-2 552 412**
**US-A-2 570 205**

(73) Titulaire: **INSTITUT FRANCAIS DU PETROLE, 4, Avenue
de Bois-Préau, F-92506 Rueil-Malmaison Cédex (FR)**

(72) Inventeur: **Mikitenko, Paul, 25, Orée de Marly,
F-78590 Noisy le Roi (FR)**
Inventeur: **Asselineau, Lionel, 115, Boulevard Bessière,
75017 Paris (FR)**

EP 0 047 204 B1

Procédé de production d'alcools déshydratés utilisables comme composants d'un carburant pour moteur.

L'invention concerne un procédé de production d'alcools déshydratés utilisables soit en tant que tels; soit comme composants d'un carburant pour moteur. Plus particulièrement, elle concerne la production d'alcools exempts d'eau par distillation extractive d'un mélange d'alcools et d'eau en présence de solvants sélectifs.

Les alcools aliphatiques de synthèse sont produits par différents procédés. On les obtient en particulier par synthèse catalytique à partir de CO et $H_2$, par oxydation partielle d'hydrocarbures ou par fermentation de jus sucrés. Le produit brut obtenu contient de petits pourcentages de sous-produits organiques (éthers, cétones, aldéhydes) ainsi que de l'eau en quantités variables, par exemple de 10 à 90% en poids.

L'utilisation d'alcools comme carburant en mélange avec l'essence nécessite des alcools déshydratés ayant une teneur résiduelle en eau généralement inférieure à 2% en poids, qu'il est impératif d'obtenir économiquement. L'emploi des alcools est proposé pour pallier la pénurie et le coût élevé, actuellement, des produits pétroliers. De plus, les alcools ont une propriété antidétonante qui permet d'éliminer en grande partie le plomb dans le super-carburant, il en résulte donc une diminution de la pollution atmosphérique. Le séchage de tels mélanges d'alcools ne peut se faire par simple distillation du fait de la formation d'azéotropes entre l'eau et les alcools aliphatiques, le méthanol excepté.

Diverses méthodes ont été proposées pour sécher principalement l'éthanol. La distillation azéotropique utilisant un hydrocarbure saturé ou aromatique ou naphténique ou un mélange de ces corps comme entraineur est utilisée dans de nombreuses installations. On forme un hétéro-azéotrope eau/hydrocarbure(s)/alcool qui distille en tête de colonne, est condensé puis partiellement recyclé et partiellement traité dans une seconde colonne; on récupère l'alcool anhydre dans la partie inférieure de la colonne. Cependant le benzène, le plus souvent utilisé dans le séchage de l'éthanol a le désavantage d'être toxique et l'apparente simplicité de la méthode est compensée par le manque de rapidité dans la séparation complète des phases aqueuse et organique de l'hétéroazéotrope.

L'utilisation du mélange n-heptane-benzène n'a pas résolu complètement le problème à cause du faible pourcentage d'eau relatif au benzène entrainé par l'hétéroazéotrope, ce qui a pour effet de réduire notablement l'efficacité du procédé (voir le brevet européen 1694).

Avec le cyclohexane, les résultats sont améliorés; cependant, on reste limité au séchage des alcools contenant 2 et 3 atomes de carbone (voir le brevet européen 1681).

On a proposé également des solvants plus lourds pour sécher l'éthanol, notamment la glycérine et les glycols, de préférence en association avec des sels ($CaCl_2$, $K_2CO_3$) de manière à augmenter suffisamment l'écart entre les volatilités relatives des alcools et de l'eau et réaliser la séparation par distillation extractive. Cependant, des difficultés apparaissent lors de la récupération des solvants, dues à la présence des sels qui forment, sous l'action de la chaleur, des gommes et des goudrons et/ou à leur décomposition. On reste limité au séchage des alcools légers, particulièrement de l'éthanol (voir le brevet russe 424 854), et les quantités de solvant d'extraction nécessaires sont souvent importantes.

Certains procédés de synthèse fournissent des mélanges d'alcools aliphatiques relativement riches en méthanol et en éthanol mais contenant également des alcools de points d'ébullition supérieurs dont la présence est bénéfique pour l'utilisation comme composants d'un carburant pour moteur.

En effet, ils améliorent en particulier la courbe de distillation et la compatibilité avec l'eau. C'est pourquoi on a avantage à utiliser les alcools en mélange plutôt qu'isolément.

On a maintenant trouvé que l'on pouvait obtenir la déshydratation d'un mélange complexe d'alcools aliphatiques renfermant au moins un alcool léger et au moins un alcool lourd en soumettant le mélange à une distillation extractive en présence de solvant sélectif suivie d'une distillation azéotropique du résidu obtenu de manière à obtenir les alcools solubles dans l'eau déshydratés, comme distillat de la première distillation, et les alcools insolubles dans l'eau dans le distillat de la deuxième distillation, sous forme d'hétéro-azéotropes dont les deux phases condensées sont ensuite distillées pour obtenir, à partir de la phase légère, les alcools lourds déshydratés et, à partir de la phase lourde, l'eau que l'on rejette.

Par alcools légers on entend les alcools ayant de 1 à 3 atomes de carbone et par alcools lourds les alcools ayant 5 et/ou 6 atomes de carbone. Des alcools ayant 4 atomes de carbone peuvent être aussi présents et considérés comme alcools légers ou lourds suivant qu'ils sortent en tête ou en queue lors de la distillation extractive, ce qui dépend de la nature du solvant utilisé.

Des alcools plus lourds ne sont pas limitatifs pour l'invention. Cependant, il est plus rare de les trouver en quantité importante dans les produits obtenus par les procédés de synthèse subcités.

Les solvants sélectifs sont définis comme ayant au moins un hétéroatome dans leur molécule et étant capables de dissoudre au moins 10% en poids d'eau ou de chacun des alcools concernés.

On peut citer, à titre d'exemples:
- Les solvants de la famille des glycols, par exemple: l'éthylèneglycol, le diéthylèneglycol, le triéthylèneglycol, le tétraéthylèneglycol, le propylèneglycol.
- Les solvants de la famille des éthers de glycol, par exemple: l'éthylèneglycol-monopropyléther, l'éthylèneglycol-monobutyléther.

– Les solvants de la famille des éthers de diéthylèneglycol, par exemple: le diéthylèneglycol-monométhyléther, le diéthylèneglycol-monoéthyléther, le diéthylèneglycol-monobutyléther, le diéthylèneglycol-diméthyléther, ou des éthers de di ou tripropylèneglycol par exemple le dipropylèneglycolméthyléther, le tripropylèneglycolméthyléther.

– Les solvants de la famille des éthanolamines, par exemple mono-, di et triéthanolamine.

– La N-méthylpyrrolidone et le sulfolane.

le procédé comporte plusieurs étapes principales:

– Une étape (a) de distillation extractive dans laquelle le mélange aqueux d'alcools aliphatiques est introduit en un point intermédiaire de la zone de distillation et le solvant en un point de cette zone situé au dessus du point d'introduction du mélange pour obtenir d'une part un produit de tête P constitué principalement par les alcools ayant de 1 à 3, parfois 4 atomes de carbone (selon la nature du solvant utilisé) dans leurs molécules (méthanol, éthanol, propanol [n et iso], certains butanols) et ne contenant pratiquement pas d'eau, ledit produit de tête étant déchargé du procédé, et, d'autre part, un produit de fond contenant pratiquement la totalité du solvant et de l'eau introduits et les alcools lourds.

– Une étape (b) de distillation azéotropique effectuée sur le produit de fond provenant de l'étape (a) dans laquelle on récupère, comme résidu, le solvant largement débarrassé d'eau, que l'on recycle à l'étape (a), et, comme distillat, le mélange hétéroazéotropique formé par l'eau et les alcools lourds que l'on condense pour obtenir sa séparation en 2 phases liquides, l'une lourde A riche en eau, l'autre légère B riche en alcools lourds dont on peut utilement recycler une partie, comme reflux, dans la colonne de distillation azéotropique et qui sont soumises chacune à des distillations dans une étape (c) de manière à obtenir, en résidus, respectivement de A une phase P contenant essentiellement l'eau, qui est rejetée, et de B une phase P′ constituée par les alcools lourds débarrassés d'eau, qui est éliminée du circuit, tandis que les distillats sont recyclés vers le mélange hétéroazéotropique condensé de l'étape (b).

On réunit les produits P et P′ pour constituer le mélange d'alcools déshydratés selon l'invention, utilisable comme alcool moteur en mélange avec de l'essence.

Dans une variante du procédé, les produits P et P′ peuvent être soumis à des distillations classiques pour produire isolément les alcools désirés.

Les mélanges d'alcools susceptibles d'être déshydratés avantageusement selon l'invention peuvent renfermer, par exemple, de 2 à 70% en poids d'eau, généralement de 15 à 50%. Ils renferment des alcools légers (méthanol, éthanol, propanols) représentant habituellement 50 à 80% en poids du mélange d'alcools proprement dit, des alcools lourds (bnutanols, pentanols, hexanols) constituant le complément. D'autres composés oxygénés ou alcools supérieurs peuvent être présents, comme indiqué plus haut.

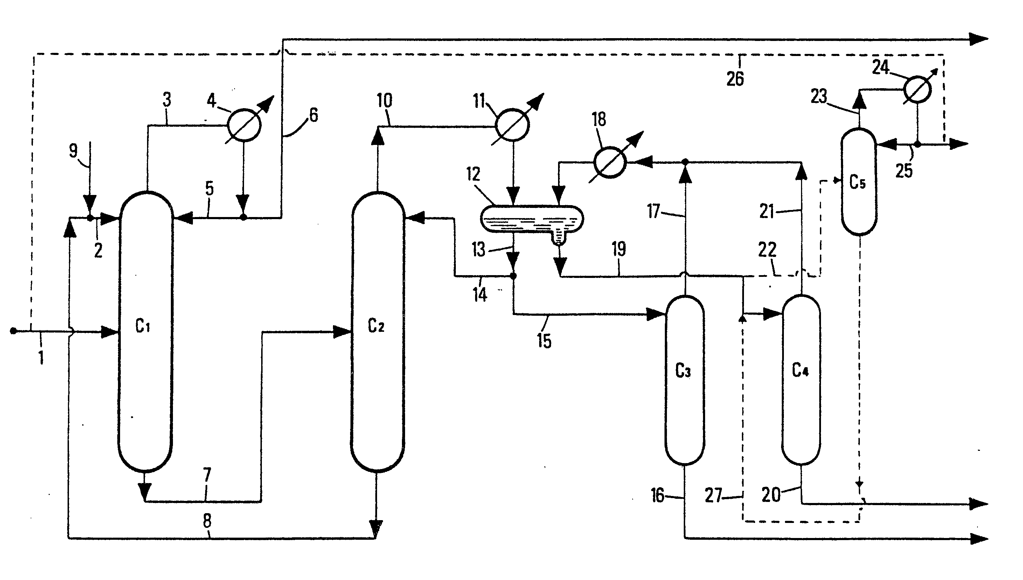

La figure annexée présente un schéma simplifié du procédé indiquant comment peut fonctionner l'installation.

Celle-ci se compose d'une colonne de distillation extractive C1 et de 3 colonnes de distillation C2, C3 et C4.

On introduit, dans la colonne C1, le mélange à traiter (eau + alcools) par la ligne 1 et le solvant d'extraction par la ligne 2. La fraction de tête de distillation passe par la ligne 3; elle est condensée dans l'échangeur 4, une partie est recyclée comme reflux par la ligne 5 et l'autre partie qui sort par la ligne 6 et qui est constituée par les alcools légers est évacuée de l'installation.

Simultanément, on récupère, par la ligne 7, une fraction contenant essentiellement du solvant, de l'eau et des alcools lourds que l'on envoie dans la colonne C2 par l'intermédiaire de cette ligne pour être séparée en ses constituants. On récupère, par ligne 8, en fond de colonne, le solvant pratiquement débarrassé d'eau qui retourne à la colonne C1, grâce à la ligne 2 (un appoint de solvant peut être fait par la ligne 9) et par la ligne 10, un distillat qui est formé par le mélange héteroazéotropique eau-alcools lourds. Après avoir été condensé dans l'échangeur 11, le distillat passe dans le décanteur 12 où il se sépare en 2 phases liquides. La phase légère, riche en alcools, est soutirée par la ligne 13; une partie est recyclée, comme reflux, par la ligne 14 et l'autre partie est dirigée, par la ligne 15, vers la colonne C3 dans laquelle ses constituants sont séparés; les alcools lourds débarrassés d'eau sont récupérés par la ligne 16 et évacués de l'installation tandis que l'eau accompagnée d'alcools lourds sort par la ligne 17 et, après avoir été condensée dans l'échangeur 18 est renvoyée dans le décanteur 12.

La phase lourde, riche en eau, est soutirée par la ligne 19 et introduite dans la colonne C4, pour obtenir, après distillation, en fond de colonne, de l'eau pratiquement pure qui sort de l'installation par la ligne 20 et, en tête de colonne, une phase renfermant des alcools lourds et de l'eau, qui par la ligne 21, va vers le condenseur 18.

Une purge de l'installation peut se faire par la ligne 22 permettant la récupération des constituants légers, tels que le méthanol, éventuellement entrainés.

Le courant soutiré est envoyé dans une colonne C5 d'où les constituants légers sortent par la ligne 23, sont condensés en 24, recyclés en partie en tant que reflux dans la colonne C5 par la ligne 25 et récupérés par la ligne 26 pour être renvoyés à la ligne 1, et l'eau entraînant de faibles quantités d'alcools sort par la ligne 27 pour être recyclée vers la ligne 19.

Exemple

Dans une colonne de distillation de 50 mm de diamètre, constituée par 8 éléments de 10 plateaux perforés avec déversoir, 1 bouilleur, un condenseur et un ballon de reflux, on introduit sur le 38è

plateau à partir du fond, un mélange d'alcools et d'eau préchauffé à 60°C, à raison de 750 g/heure.

La composition détaillée suivant le nombre de carbones de la molécule de ces alcools est donnée dans la deuxième colonne du tableau. Sur le 74è plateau on introduit avec un débit de 2250 g/h du diéthylèneglycol qui provient du fond de la colonne de régénération du solvant et qui contient en moyenne 300 ppm d'eau et 0,8% en poids d'alcools, principalement de l'hexanol (composition donnée dans la troisième colonne du tableau).

La marche de la colonne est réglée de manière à sortir la quasi totalité de l'eau en fond de colonne, en établissant un reflux de distillat par rapport à la charge égal à 0,65:1.

Les vapeurs de tête de colonne sont condensées et envoyées dans le ballon de reflux; une partie en est recyclée à la colonne suivant le taux de reflux indiqué ci-dessus tandis que l'autre partie qui constitue la production d'alcools légers déshydratés est évacuée du système. La composition pondérale de cette fraction alcools légers est donnée dans la 4è colonne du tableau.

Du fond de la colonne, on soutire, par une vanne commandée par un lecteur de niveau de liquide dans le bouilleur, un mélange qui est envoyé dans un bac tampon puis, par l'intermédiaire d'une pompe, sur le 30è plateau de la colonne de régénération de solvant, qui est identique à la première mais constituée de 5 éléments de 10 plateaux. Cette seconde colonne est réglée de manière à laisser moins de 500 ppm d'eau dans le solvant régénéré, qui sort en fond à 242°C et est recyclé dans la première colonne comme indiqué précédemment, après passage dans un bac tampon. Les vapeurs de tête qui sont à une température de 97°C sont condensées et envoyées dans un ballon de reflux qui joue également le rôle de décanteur pour séparer la phase riche en alcools de la phase riche en eau. Une partie de la phase alcools, soit 350 cm³/h est envoyée en reflux au sommet de la colonne tandis que l'autre partie est envoyée au sommet d'une colonne de 30 plateaux, dont la chauffe est réglée de manière à produire en fond les alcools lourds avec une teneur en eau ⩽0,2% poids. Les vapeurs de tête de cette colonne sont condensées et renvoyées dans le ballon de reflux-décanteur de la colonne de régénération de solvant.

La phase aqueuse issue de ce ballon de reflux-décanteur est envoyée dans la partie supérieure d'une quatrième colonne, constituée de 30 plateaux dont la chauffe est réglée de manière à produire en fond de l'eau pratiquement exempte d'alcools, qui est évacuée du système. Les vapeurs de tête de cette colonne sont condensées et envoyées dans le ballon de reflux-décanteur de la colonne de régénération de solvant. Les compositions pondérales moyennes des produits sont présentées dans les colonnes 5 et 6 du tableau.

Tableau 1
Composition des entrées – Sorties du systeme (% en poids)

| Nature des composés | Charge colonne | Solvant colonne | Alcools légers | Alcools lourds | Eau |
|---|---|---|---|---|---|
| Méthanol | 26,8 | – | 40,8 | – | – |
| Ethanol | 27,7 | – | 42,2 | – | – |
| Propanols | 10,5 | – | 15,8 | 0,9 | – |
| Butanols | 5,8 | – | 1,0 | 35,6 | – |
| Pentanols | 6,7 | – | – | 46,2 | Traces |
| Hexanols | 2,5 | 0,8 | – | 17,2 | Traces |
| Eau | 20 | 300 ppm | 0,2 | 0,1 | >99,8 |
| Diéthylène-glycol | – | 99,1 | – | – | – |

## Revendications

1. Procédé de production d'alcools aliphatiques déshydratés à partir d'un mélange d'alcools aliphatiques et d'eau, ledit mélange contenant au moins un alcool ayant de 1 à 3 atomes de carbone et au moins un alcool ayant 5 ou 6 atomes de carbone, ledit procédé comprenant les étapes suivantes:

a) on introduit le mélange alcools-eau dans une 1è zone de fractionnement, en un point intermédiaire de celle-ci,

b) on introduit un solvant sélectif en un point de la 1è zone de fractionnement plus élevé que le point d'introduction du mélange,

c) on soutire en tête un effluent vapeur contenant les alcools aliphatiques légers déshydratés,

d) on soutire en fond une phase liquide riche en solvant sélectif et contenant l'eau et les alcools lourds,

e) on introduit ladite phase liquide dans une 2è zone de fractionnement,

f) on soutire en tête de la 2è zone de fractionnement un effluent vapeur constitué par un mélange hétéroazéotropique eau et alcools lourds,

g) on soutire en fond de la 2è zone de fractionnement une phase liquide constituée par le solvant sélectif largement débarrassé d'eau et on le renvoie à l'étape (b),

h) on condense le mélange hétéroazéotropique de l'étape (f) en 2 phases liquides, une phase légère riche en alcools et une phase lourde enrichie en eau, et on sépare ces phases,

i) on soutire la phase légère obtenue à l'étape (h) et on en envoie au moins une fraction dans une 3è zone de fractionnement,

j) on soutire en tête de la 3è zone de fractionnement un effluent vapeur et on le renvoie à l'étape (h),

k) on soutire en fond de la 3è zone de fractionnement une phase liquide constituée par les alcools aliphatiques lourds déshydratés,

l) on soutire la phase lourde obtenue à l'étape (h) et on en introduit au moins une partie dans une 4ème zone de fractionnement,

m) on retire en tête de la 4è zone de fractionnement un effluent vapeur et on le renvoie à l'étape (h), et

n) on récupère en fond de la 4è zone de fractionnement une phase liquide constituée essentiellement par de l'eau.

2. Procédé selon la revendication 1, dans lequel a) on prélève la fraction restante de la phase lourde de l'étape (h) et on l'introduit dans une 5ème zone de fractionnement,,

b) on soutire en tête de la 5è zone de fractionnement un effluent vapeur et on le renvoie à l'étape (a),

c) on récupère en fond de la 5è zone de fractionnement une phase liquide et on la renvoie à l'étape (1).

3. Procédé selon l'une des revendications 1 ou 2 dans lequel le mélange d'alcools aliphatiques et d'eau est constitué d'alcools ayant 1 à 6 atomes de carbone.

4. Procédé selon l'une des revendications 1 à 3 dans lequel le solvant sélectif est un glycol ou un éther de glycol.

5. Procédé selon l'une des revendications 1 à 3 dans lequel le solvant sélectif est une éthanolamine.

6. Procédé selon l'une des revendications 1 à 3 dans lequel le solvant sélectif est la N-méthylpyrolidone.

7. Procédé selon l'une des revendications 1 à 3 dans lequel le solvant sélectif est le sulfolane.

8. Procédé selon l'une des revendications 1 à 7 dans lequel le mélange d'alcools et d'eau contient de 2 à 50% en poids d'eau.

9. Procédé selon l'une des revendications 1 à 7 dans lequel le mélange d'alcools et d'eau contient de 15 à 25% en poids d'eau.

10. Utilisation des alcools aliphatiques déshydratés, obtenus par le procédé de l'une quelconque des revendications 1 à 9, comme composant d'un carburant pour moteur.

**Patentansprüche**

1. Verfahren zur Herstellung von entwässerten aliphatischen Alkoholen ausgehend von einer Mischung der aliphatischen Alkohole mit Wasser, wobei diese Mischung mindestens einen Alkohol mit 1 bis 3 Kohlenstoffatomen und mindestens einen Alkohol mit 5 oder 6 Kohlenstoffatomen enthält, und wobei dieses Verfahren folgende Stufen aufweist:

a) man führt die Mischung von Alkohol und Wasser in eine erste Fraktionierzone an einem Punkt in der Mitte derselben ein,

b) man führt ein selektives Lösungsmittel an einem Punkt der ersten Fraktionierzone ein, der höher ist als der Punkt der Einführung der Mischung,

c) man zieht am Kopf einen dampfförmigen Abstrom ab, der die entwässerten leichten aliphatischen Alkohole enthält,

d) man zieht am Boden eine flüssige Phase ab, die reich an selektivem Lösungsmittel ist und Wasser und die schweren Alkohole enthält,

e) man führt diese flüssige Phase in eine zweite Fraktionierzone ein,

f) man zieht am Kopf der zweiten Fraktionierzone einen dampfförmigen Abstrom ab, der aus einem heteroazeotropen Gemisch von Wasser und schweren Alkoholen besteht,

g) man zieht am Boden der zweiten Fraktionierzone eine flüssige Phase ab, die aus dem selektiven Lösungsmittel besteht, das an Wasser grossenteils angereichert ist und führt sie zur Stufe b) zurück,

h) man kondensiert das heteroazeotrope Gemisch der Stufe f) zu zwei flüssigen Phasen, einer leichten Phase, die an Alkoholen reich ist und einer schweren Phase, die an Wasser angereichert ist und trennt diese Phasen

i) man zieht die in Stufe h) erhaltene leichte Phase ab und überführt zumindest eine Fraktion davon in eine dritte Fraktionierzone

j) man zieht am Kopf der dritten Fraktionierzone einen dampfförmigen Abstrom ab und führt ihn zur Stufe h) zurück,

k) man zieht am Boden der dritten Fraktionierzone eine flüssige Phase ab, die aus den entwässerten schweren aliphatischen Alkoholen besteht,

l) man zieht die in Stufe h) erhaltene schwere Phase ab und führt zumindest einen Teil davon in eine vierte Fraktionierzone ein,

m) man zieht am Kopf der vierten Fraktionierzone einen dampfförmigen Abstrom ab und führt ihn zur Stufe h) zurück, und

n) man gewinnt am Boden der vierten Fraktionierzone eine flüssige Phase, die im wesentlichen aus Wasser besteht.

2. Verfahren nach Anspruch 1, worin

a) man die verbleibende Fraktion der schweren Phase der Stufe h) abnimmt und sie in eine fünfte Fraktionierzone einführt,

b) man am Kopf der fünften Fraktionierzone einen dampfförmigen Abstrom abzieht und ihn zur Stufe a) zurückführt,

c) man am Boden der fünften Fraktionierzone eine flüssige Phase gewinnt und sie zur Stufe 1) zurückführt.

3. Verfahren nach einem der Ansprüche 1 oder 2, worin die Mischung der aliphatischen Alkohole mit Wasser aus Alkoholen mit 1 bis 6 Kohlenstoffatomen besteht.

4. Verfahren nach einem der Ansprüche 1 bis 3, worin das selektive Lösungsmittel ein Glykol oder ein Glykoläther ist.

5. Verfahren nach einem der Ansprüche 1 bis 3, worin das selektive Lösungsmittel ein Ethanolamin ist.

6. Verfahren nach einem der Ansprüche 1 bis 3, worin das selektive Lösungsmittel N-Methylpyrrolidon ist.

7. Verfahren nach einem der Ansprüche 1 bis 3, worin das selektive Lösungsmittel Sulfolan ist.

8. Verfahren nach einem der Ansprüche 1 bis 7, worin die Mischung von Alkoholen und Wasser 2 bis 50 Gew.-% Wasser enthält.

9. Verfahren nach einem der Ansprüche 1 bis 7, worin die Mischung von Alkoholen und Wasser 15 bis 25 Gew.-% Wasser enthält.

10. Verwendung der entwässerten aliphatischen Alkohole, erhalten nach dem Verfahren einer der Ansprüche 1 bis 9, als Bestandteil eines Motortreibstoffs.

## Claims

1. A process for producing dehydrated aliphatic alcohols from a mixture of aliphatic alcohols and water, said mixture containing at least one alcohol having 1 to 3 carbon atoms and at least one alcohol having 5 or 6 carbon atoms, said process comprising the following steps:

a) introducing the alcohols – water mixture into a first fractionation zone at an intermediary point thereof,

b) introducing a selective solvent at a point of the first fractionation zone above the point of introduction of the mixture,

c) withdrawing at the top a vapor effluent containing the dehydrated light aliphatic alcohols,

d) withdrawing from the bottom a liquid phase of high selective solvent content and containing water and heavy alcohols,

e) introducing said liquid phase into a second fractionation zone,

f) withdrawing at the top of the second fractionation zone a vapor effluent consisting of an hetero-azeotropic mixture of water with heavy alcohols,

g) withdrawing from the bottom of the second fractionation zone a liquid phase consisting of the selective solvent freed to a large extent of water and feeding it back to step (b),

h) condensing the hetero-azeotropic mixture of step (f) into two liquid phases, a light phase of high alcohol content and a heavy phase of increased water content and separating these two phases

i) withdrawing the light phase obtained in step (h) and feeding at least a fraction thereof into a third fractionation zone,

j) withdrawing at the top of the third fractionation zone a vapor effluent and feeding it back to step (h),

k) withdrawing from the bottom of the third fractionation zone a liquid phase consisting of dehydrated heavy aliphatic alcohols,

l) withdrawing the heavy phase obtained in step (h) and introducing at least a portion thereof into a fourth fractionation zone,

m) withdrawing at the top of the fourth fractionation zone a vapor effluent and feeding it back to step (h), and

n) recovering from the bottom of the fourth fractionation zone a liquid phase consisting essentially of water,

2. A process according to Claim 1 comprising

a) withdrawing a remainder portion of the heavy phase of step (h) and introducing it into a fifth fractionation zone,

b) withdrawing, at the top of the fifth fractionation zone, a vapor effluent and feeding it back to step (a),

c) recovering from the bottom of the fifth fractionation zone a liquid phase and feeding it back to step (1).

3. A process according to one of Claims 1 to 2, where the mixture of aliphatic alcohols and water comprises alcohols having from 1 to 6 carbon atoms.

4. A process according to one of Claims 1 to 3, wherein the selective solvent is a glycol or a glycol ether.

5. A process according to one of Claims 1 to 3, wherein the selective solvent is an ethanolamine.

6. A process according to one of Claims 1 to 3, wherein the selective solvent is N-methylpyrrolidone

7. A process according to one of Claims 1 to 3, wherein the selective solvent is sulfolane.

8. A process according to one of Claims 1 to 7, wherein the mixture of alcohols and water contains from 2 to 50% by weight of water.

9. A process according to one of Claims 1 to 7, wherein the mixture of alcohols and water contains from 15 to 25% by weight of water.

10. The use of dehydrated aliphatic alcohols obtained by the process according to any of claims 1 to 9, as component for motor fuel.

0 047 204